# EUROPEAN PATENT APPLICATION

(11) **EP 4 617 702 A1**
(43) Date of publication of application: **17.09.2025**
(21) Application number: 24163235.5
(22) Date of filing: 13.03.2024
(51) Int. Cl.: G01R 33/563, G01R 33/565

(54) **EFFICIENT MAGNETIC RESONANCE ELASTOGRAPHY USING INTERLEAVED MOTION ENCODING**

(71) Applicant: Siemens Healthineers AG, 91301 Forchheim (DE); Centre National de la Recherche Scientifique (CNRS), 75016 Paris Cedex 16 (FR); INSTITUT NATIONAL DE LA SANTE ET DE LA RECHERCHE MEDICALE - INSERM, 75013 Paris (FR); King's College London, London WC2R 2LS (GB); Université Paris XIII Paris-Nord, 93430 Villetaneuse (FR); Université Paris Cité, 75006 Paris (FR)
(72) Inventor: TRIPP, Donovan, SE1 7AL London (GB); DARWISH, Omar, SE8 5EP London (GB); SINKUS, Ralph, 95620 Parmain (FR); NEJI, Radhouene, N65XP London (GB)
(74) Representative: Siemens Healthineers Patent Attorneys

(57) **Abstract**

Magnetic Resonance Elastography (MRE) shall be performed more efficient. Therefore, there is proposed a method including the steps of providing a periodical vibration signal (11) for exciting mechanical vibrations within an object (8) to be examined with a vibration period, characterized by
- sampling the vibration signal (11) with a sampling period (wp1, wp2, wp3, wp4), the sampling period (wp1, wp2, wp3, wp4) corresponding to a natural number including zero of vibration periods plus a fixed time delay (13),
- the fixed time delay multiplied with a sampling number is equal to the vibration period, the sampling number being a natural number greater than two,
- three motion encoding gradients (MEGₓ, MEG_{y}, MEG_{z}) are performed for magnetic resonance acquisition in each sampling period (wp1, wp2, wp3, wp4).

## Description

The present invention relates to a method of performing Magnetic Resonance Elastography. Furthermore, the present invention relates to a Magnetic Resonance Elastography device and a computer program for controlling such device.

Independent of the grammatical term usage, individuals with male, female or other gender identities are included within the term.

A fundamental paper of Magnetic Resonance Elastography (MRE) is Muthupillai R, Lomas DJ, Rossman PJ, Greenleaf JF, Manduca A, Ehman RL. Magnetic resonance elastography by direct visualization of propagating acoustic strain waves. Science. 1995 Sep 29;269(5232):1854-7.

MRE enables non-invasive estimation of biomechanical tissue properties in-vivo allowing in-vivo estimation of biomechanical properties in tissues, with applications including the assessment and staging of liver fibrosis as shown in Venkatesh, S.K., Yin, M. and Ehman, R.L. (2013), Magnetic resonance elastography of liver: Technique, analysis, and clinical applications. J. Magn. Reson. Imaging, 37: 544-555.

The simultaneous assessment of liver fibrosis and inflammation from elasticity and viscosity has previously been demonstrated with 3D MRE sequences by Sinkus, R, Lambert, S, Abd-Elmoniem, KZ, et al. Rheological determinants for simultaneous staging of hepatic fibrosis and inflammation in patients with chronic liver disease. NMR in Biomedicine. 2018; 31:e3956 and by Darwish OI, Gharib AM, Jeljeli S, Metwalli NS, Feeley J, Rotman Y, Brown RJ, Ouwerkerk R, Kleiner DE, Stäb D, Speier P, Sinkus R, Neji R. Single Breath-Hold 3-Dimensional Magnetic Resonance Elastography Depicts Liver Fibrosis and Inflammation in Obese Patients. Invest Radiol. 2023 Jun 1;58(6):413-419.

MRE reconstruction using the curl operator to remove the compressional wave was performed by Sinkus, R., Tanter, M., Catheline, S., Lorenzen, J., Kuhl, C., Sondermann, E. and Fink, M. (2005), Imaging anisotropic and viscous properties of breast tissue by magnetic resonance-elastography. Magn. Reson. Med., 53: 372-387.

Current volumetric 3D MRE sequences are typically acquired in 2D slices, missing out on the higher SNR afforded by a 3D acquisition.

Published 3D MRE techniques are based on wave offset interleaving like van Schelt AS, Gottwald LM, Wassenaar NPM, Runge JH, Sinkus R, Stoker J, Nederveen AJ, Schrauben EM. Single Breath-Hold MR Elastography for Fast Biomechanical Probing of Pancreatic Stiffness. J Magn Reson Imaging. 2023 May 17. doi: 10.1002/jmri.28773. Epub ahead of print. PMID: 37194646.

A generalized multi-shot MRE sequence is shown by Guenthner C, Sethi S, Troelstra M, Dokumaci AS, Sinkus R, Kozerke S. Ristretto MRE: A generalized multi-shot GRE-MRE sequence. NMR Biomed. 2019 May;32(5):e4049. doi: 10.1002/nbm.4049. Epub 2019 Jan 29. PMID: 30697827; PMCID: PMC6590281.

A minimum cost flow technique for unwrapping phase images is presented in Costantini M. A novel phase unwrapping method based on network programming. IEEE Transactions on Geoscience and Remote Sensing. 1998;36(3):813-821. doi:10.1109/36.673674.

The object of the present invention is to provide an efficient acquisition scheme for Magnetic Resonance Elastography. Specifically, a respective method for performing MRE and a respective MRE device shall be provided.

According to the present invention this object is solved by a method and a Magnetic Resonance Elastography device according to the independent claims. Favorable further developments are defined in the subclaims.

A method of performing Magnetic Resonance Elastography is therefore proposed including the step of providing a periodical vibration signal for exciting mechanical vibrations with a vibration period. This means that elastography based on magnetic resonance tomography is performed. A periodical vibration signal is provided as basis for the elastography. This periodical vibration signal can serve for exciting mechanical vibrations. For instance, a electromechanical transducer can be supplied with a periodical vibration signal.

In a further step the vibration signal is sampled with a sampling period. The sampling period is also called "wave phase" in this document. The sampling period corresponds to a natural number including 0 of vibration periods plus a fixed delay. For instance, the sampling period is equal to 2,25 vibration periods. Thus, the natural number of vibration periods is two and the fixed delay is a quarter of a vibration period. According to another example the sampling period may also be equal to 0,125 vibration periods, which means that the natural number of the vibration period is 0 and the fixed delay is 1/8 of a vibration period. The number of vibration periods within one sampling period can also be significantly larger.

The fixed delay multiplied with a sampling number is equal to the vibration period, wherein the sampling number is a natural number greater than 2. This means that the vibration signal is sampled more than two times in one vibration period. For instance, a vibration signal is sampled 3, 4, 5 etc. times within one vibration period. The fixed delay ensures a certain phase shift of the sampling period with respect to the vibration period. After a number of sampling periods corresponding to the sampling number the phase shift between the vibration period and the sampling period is 0. Thus, the acquisition can be continued with a new sampling period without time delay.

Three motion encoding gradients (and optional a reference scan) are performed for magnetic resonance equisition in each sampling period. This means that the sampling period, i.e. the wave phase, is divided into four time slots. Three time slots are used for applying motion encoding gradients. Preferably, the gradients are different with respect to spatial directions. A fourth time slot is used for a reference scan. The reference scan can be used to standardize other measurement scans.

The advantage of the inventive method is that a very efficient scheme is provided compared to other known techniques of the prior art.

According to a specific embodiment the motion encoding gradients are part of a 3D slab-selective Magnetic Resonance Imaging sequence. The slab-selective stimulation includes a stimulation within a slice as well as a stumulation perpendicular to the slice.

In another embodiment the motion encoding gradients are applied at different phases of the vibration signal and a corresponding phase correction is applied to each motion encoding by the respective one of the motion encoding gradients. If the interleaved motion encoding gradients are applied at different time points, i.e. different phases, a respective phase correction has to be applied to each motion encoding. Preferrably the phase correction is performed after temporal Fourier transform followed by an optional subtraction of a reference background phase.

In a further embodiment Magnetic Resonance Imaging reconstruction is performed based on signals obtained from the applied motion encoding gradients. Thus, standard MRI reconstruction techniques can be used for MRE.

According to still another embodiment a result from the reconstruction is used for evaluating stiffness and/or viscosity of an object being examined by the MRE. Such evaluation can be performed by a user or automatically (e.g. by image processing).

In one embodiment the natural number of vibration periods within one sampling period is equal to one, two or three. This means that the motion encoding gradients and the reference scan have to be provided/performed within one, two or three vibration periods plus the respective fixed delay. This further means that the sampling period and the vibration period are of the same order of magnitude. Otherwise, if a comparatively high vibration frequency is necessary, the number of vibration periods within one sampling period may also be higher than three.

In another embodiment the fixed delay corresponds to one third, one quarter or one fifth of the vibration period. This means that the phase shift between vibration signal and sampling period is zero after three, four or five sampling periods. This also means that the vibration period is sampled three, four or five times. Such sampling rate is sufficient for obtaining meaningful scans.

According to another embodiment the sampling period comprises four time slots, one of the four time slots for each of the three motion encoding gradients and one time slot for the reference scan as already indicated above. Thus, the gradients are not interleaved but applied successively one after another. After the application of the three motion encoding gradients the reference scan is performed. Consequently, a reference scan is performed in each sampling period. Thus, the MRE data acquisition is more reliable.

In a favorable development of the before mentioned scheme the four time slots are the same size. Thus, e.g. an analog-digital converter can be used for clocking the MRE scans.

According to a further embodiment the three motion encoding gradients represent gradients in three orthogonal space directions. Specifically, there may be applied an x-gradient, a y-gradient and a z-gradient successively. Thus, a total 3D volume can be stimulated by the motion encoding gradients.

In another embodiment no motion encoding gradient is applied during the reference scan. As explained above, the reference scan shall provide reference data for standardizing the other measurements. Thus, a large number of scans can be related to each other.

In still another embodiment the above described method is repeated as k-space poit acquisition step for each k-space point of a k-space. In other words, the specific acquisition scheme with the sampling period aligned with a vibrational signal as defined above is applied for each k-space point of the k-space. At the end of the acquisition of the complete k-space the data are back transformed in order to obtain the MRE signal of a sample.

According to a favorable further development of the inventive method the k-space point acquistion steps are synchronized with a vibrational signal. The synchronization is realized by the fact that a multiple of the fixed delay corresponds to the vibrational period and the multiple itself is equal to the sampling number for sampling one vibrational period.

In a preferred embodiment the vibrational signal has a frequency in the range of 20 Hz to 200 Hz. For example, the frequency of the vibrational signal is 60 Hz. Such vibration frequency causes proper share waves in samples like human tissue in order to gain meaningful MRE signals.

The above object is also solved by a Magnetic Resonance Elastography device comprising signal generating means for providing a periodical vibration signal for exciting mechanical vibrations (within an object to be examined) with a vibration period, sampling means for sampling the vibration signal with a sampling period (corresponding to one wave phase), the sampling period corresponding to a natural number including zero of vibration periods plus a fixed delay, wherein the fixed delay multiplied with a sampling number is equal to the vibration period, the sampling number being a natural number greater than two, and gradient application means for applying in each sampling period: one motion encoding gradient each in three motion encoding scans and no motion encoding gradient in one reference scan.

The advantages and further developments of the above-described method also apply to the Magnetic Resonance Elastography device analogously. Thus, the mentioned method steps can be regarded as functional features of the device.

Furthermore, according to the present invention there is provided a computer program product comprising instructions which, when the program is executed by a Magnetic Resonance Elastography device as described above, cause the Magnetic Resonance Elastography device to carry out one of the above defined methods. Additionally, a computer-readable medium comprising instructions which, when executed by a Magnetic Resonance Elastography device as mentioned above, cause the Magnetic Resonance Elastography device to carry out one of the above defined methods.

The present invention will now be described in more detail along with the attached drawings showing in:
FIG 1 a schematic diagram of a MRE device;
FIG 2 a timing diagram for the acquisition of all readouts at one phase encoding with the proposed sequence;
FIG 3 A: curl in z
   B: magnitude of reference imaging without motion encoding
   C: total wave amplitude
   D: shear wave speed Cs
   E: magnitude of the complex-valued shear modulus |G*|
   F: loss modulus G"

The following embodiments represent favorable examples of the present invention.

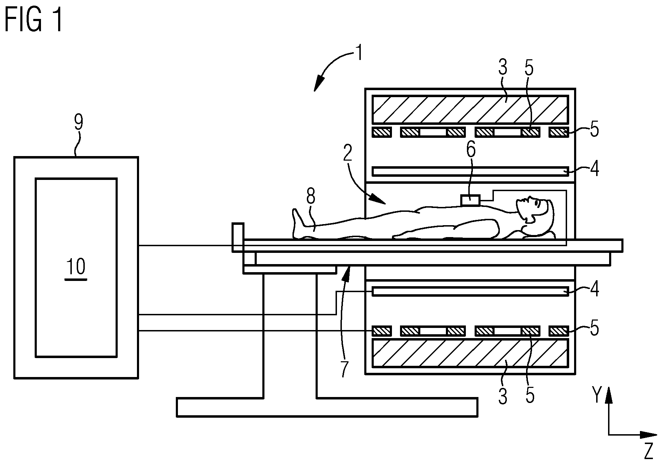

FIG 1 shows a schematic representation of an exemplary implementation of an MRE-system 1 according to the invention.

The MRE-system comprises a magnet unit with an imaging region 2, for example within a patient tunnel for placing an object 8, in particular a patient, to be imaged. The magnet unit comprises a field magnet 3 that generates a main magnet field for aligning nuclear spins of the object, in particular within the imaging region 2. The imaging region 2 is characterized by a very homogeneous static main magnet field, the homogeneity relating, in particular, to the magnetic field strength. The field magnet 3 may, for example, be a superconducting magnet capable of providing magnetic fields with a magnetic flux density in the order of several Tesla, in particular in the order of 7 T or more. A patient table 7 may be movable within the patient tunnel.

Furthermore, the magnet unit comprises a gradient coil arrangement 5 with several gradient coils that are designed to superimpose location-dependent magnetic fields in the three spatial directions on the static main magnet field for spatial differentiation and, in particular, slice selection. The gradient coils of the gradient coil arrangement 5 may, for example, be designed as coils of normal conducting wires, which may, for example, generate mutually orthogonal fields or field gradients in the recording region.

The MRE-system 1, in particular the magnet unit, comprises a transmission coil arrangement, which contains one or more RF-coils 4. It is noted that the one or more RF-coils 4 of the transmission coil arrangement may, depending on the specific implementation or application, also be used as receiving coils. Optionally, the MRI-system 1 may also comprise one or more local coils (not shown in FIG 1), which may be arranged in the immediate vicinity of the object 8, for example on the object 8 or in the patient table 7. The local coils may serve as receiving coils and/or transmission coils.

Furthermore, the MRE-system comprises a (pulse) wave generator 6 for exciting shear waves within the object/patient to be examined.

The MRE-system 1 also comprises a data processing apparatus 9 including at least one computing unit 10. The at least one computing unit 10 is configured to carry out a computer-implemented method for controlling the MRE-system according to a specific timing scheme. Particularly, the time control of the wave generator 6 is synchronized with the MR imaging. As a result of the computer-implemented method, the at least one computing unit 10 controlls the of the MRE-system so that the acquisition is performed in a very efficient way.

In response to the excitation RF-pulses, the at least one computing unit 10 receives corresponding NMR-signals from the receiving coils and may generate respective MR-images of the object 8 depending on those signals.

In particular, the at least one computing unit may comprise a readout control unit, which is connected to the at least one RF-coil 4 and/or the local coil. Depending on the detected MR-signals, the readout control unit, which may comprise an analogue-to-digital converter, ADC, may generate corresponding MR-data, in particular in k-space.

The at least one computing unit 10 may evaluate the MR-data and, for example, carry out a two-dimensional or three-dimensional image reconstruction based on the MR-data. The at least one computing unit 10 also comprises a sending control unit, which is connected to and controls the RF-coil(s) 4 and/or the local coil to emit the excitation RF-pulses and, for example refocusing RF-pulses and other RF-pulses. The at least one computing unit 10 comprises gradient control unit, which is connected to and controls the gradient coil arrangement 5 to apply, for example, slice selecting gradients, gradients for frequency and/or phase encoding, defocusing gradients and/or readout gradients and so forth. Additionally, the at least one computing unit 10 comprises a wave generation control unit adapted to control the wave generator.

It is noted that the described structure of the MRI-system 1 is a non-limiting example only. The different required tasks and functions may also be distributed differently and/or to different units in other applications.

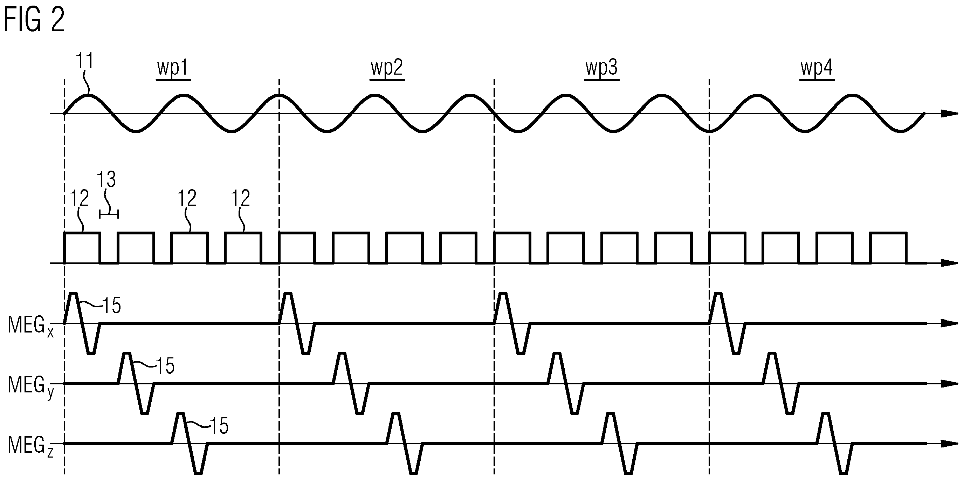

The design of a proposed 3D MRE sequence with 3D slab-selective phase-contrast spoiled gradient echo acquisition is shown in FIG 2.

The acquisition of MRE data is performed in the k-space. For each k-space point of the k-space the acquisition scheme of FIG 2 is performed. This acquisition scheme comprises an outer loop and an inner loop. The outer loop results from the sampling of vibration wave 11 (shear wave). In the case of FIG 2 the vibration wave 11 is sampled four times in one period. Thus, four waves phases wp1, wp2, wp3 and wp4 result. In other words, the vibration wave 11 is sampled each quarter wave length. The frequency of the vibration wave 11 may be 60 Hz. However, the proposed technique is not limited to this frequency. Any other appropriate frequency may be used.

In the preset example the 3D acquistion of one sampling point takes e.g. 37,5 ms which corresponds to two and a quarter wave length of a 60 Hz vibration wave.

Within each wave phase wp1, wp2, wp3 and wp4 an inner acquistion loop is performed. The inner acquisition loop comprises four time slots 12. Each time slot 12 is separated from the next time slot 12 by a time delay 13. Specifically, in this example each wave phase wp1, wp2, wp3 and wp4 comprises four time slots 12 each followed by a time delay 13. Thus, the timing signal including the time slots 12 and the time delays 13 is a periodical signal with phase Phi = 0 at each sampling point of the vibration wave 11.

Each inner acquisition loop includes three motion encodings and a reference scan without motion encoding. Specifically, a motion encoding gradient MEGₓ is applied in the first time slot 12, a second motion encoding gradient MEG_{y} is applied in the second time slot 12 annd a third motion encoding gradient MEG_{z} is applied in the third time slot 12. No motion encoding gradient is applied during the fourth time slot 12, which is used for the reference scan. The constant delay at the end of each imaging readout shifts the acquisition to the successive wave offset, similar to the MRE sequence of Ristretto et al. mentioned in the introductory part of this document. As a consequence, the total acquisition time is minimized.

This acquisition scheme of FIG 2 is repeated for all wave phases wp1, wp2, wp3 and wp4 until all wave offsets are acquired. Then the next k-space point is acquired in a similar manner.

Advantageously each k-space point acquisition is synchronised with the mechanical vibration. An important aspect is that for each motion encoding step, the design of the sequence allows to measure all k-space points in the 2D phase-encoding plane at the same wave motion state, thereby avoiding motion related ghosting artifacts.

The sequence can be combined with any 2D phase encoding acceleration technique.

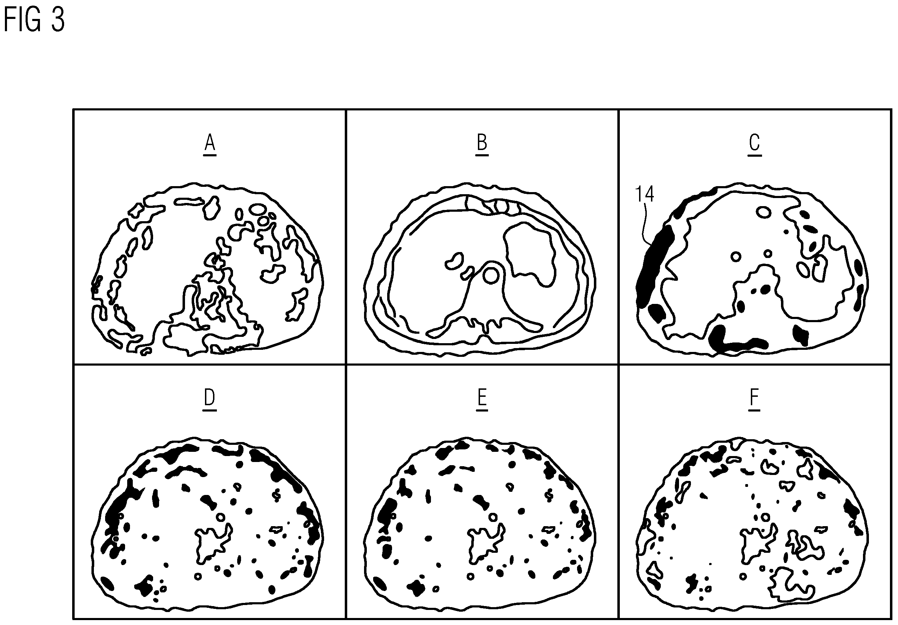

The proposed sequence cab be implemented on a 3T system to realise a 3D slab-selective (e.g. liver) MRE measurement in a single breath-hold. Experiments were performed with 60Hz actuation frequency with four wave-phase offsets and a four-point unbalanced motion encoding scheme. A breath-hold duration of 22 seconds was necessary. The proposed sequence was validated for a liver acquisition in one healthy subject as shown in FIG 3.

The obtained phase images may be unwrapped using the minimum cost flow technique as stated by Constantini. The n-th motion encoding step (n = 1..3) in the proposed sequence is acquired at a different time with respect to the 0-th motion encoding step and therefore accumulates a phase φₙ=2πn φ_{inc} where φ_{inc}=(N_{Tvib} )/(4 N_{wp} ), where N_{Tvib} is the number of vibration periods required for the acquisition of all wave offsets and motion encodings for one k-space point and N_{wp} is the number of acquired wave offsets. Therefore, to obtain the 3D displacement field, a phase correction scheme is applied to each motion encoding after the temporal Fourier transform followed by the subtraction of the reference background phase. A 3D Gaussian filter of width σ = 2 pixels and a support of 3x3x3 pixels was applied to the displacement field. MRE reconstruction was performed using the curl operator to remove the compressional wave followed by a direct inversion of the complex wave equation as proposed by Sinkus et. al. The following parameters were retrieved: the magnitude of the complex-valued shear modulus (|G*| [kPa]), the shear wave speed (Cs [m/s]), and the loss modulus (G" [kPa]). Respective results are shown in FIG 3. Picture A of FIG 3 is the calculated curl of the wave field in the Z direction of the object (here liver). This can represent the propagation of the shear wave in the liver/object. Picture B of FIG 3 represents the magnitude of the reference scan without motion encoding. The picture corresponds to a usual MRT image. Picture C of FIG 3 shows the total wave amplitude. The black regions 14 reflect higher amplitude of vibrations. Pictures D to F of FIG 3 show viscoelastic parameters to explain the behavior/state of the tissue. Picture D represents the shear wave speed Cs. Cs describes how stiff tissue is. Fibrotic liver would be stiff and would be recognizable by elevated Cs. Picture E of FIG 3 shows the magnitude of the complex valued shear modulus |G*|, which is correlated with the speed Cs. |G*| displays how stiff a tissue is (e.g. fibrotic liver is stiff). Picture F of FIG 3 shows the loss modulus G". It it describes the viscous behavior of the tissue (for example honey is very viscous) and research argues that inflamed liver is more viscus.

Compared to previously published 3D MRE techniques, the above embodiments allow a more efficient scheme than the method of van Schelt that is based on wave offset interleaving that requires a substantial increase in TR and an acquisition of five wave offsets instead of four. The present technique may also be based on a cartesian trajectory, which is less sensitive to eddy currents and off-resonance effects than a 3D fast MRE sequence based on a spiral trajectory.

## Claims

1. Method of performing Magnetic Resonance Elastography including the steps of
- providing a periodical vibration signal (11) for exciting mechanical vibrations with a vibration period,
**characterized by**
- sampling the vibration signal with a sampling period (wp1, wp2, wp3, wp4), the sampling period (wp1, wp2, wp3, wp4) corresponding to a natural number including zero of vibration periods plus a fixed delay (13),
- the fixed delay (13) multiplied with a sampling number is equal to the vibration period, the sampling number being a natural number greater than two,
- three motion encoding gradients (MEGₓ, MEG_{y}, MEG_{z}) are applied for magnetic resonance acquisition in each sampling period (wp1, wp2, wp3, wp4).

2. Method according to claim 1, wherein the motion encoding gradients (MEGₓ, MEG_{y}, MEG_{z}) are part of a 3D slab-selective Magnetic Resonance Imaging sequence.

3. Method according to claim 1 or 2, wherein the motion encoding gradients (MEGₓ, MEG_{y}, MEG_{z})are applied at different phases of the vibration signal and a corresponding phase correction is applied to each motion encoding by the respective one of the motion encoding gradients (MEGₓ, MEG_{y}, MEG_{z})

4. Method according to one of the preceding claims, wherein Magnetic Resonance Imaging reconstruction is performed based on signals obtained from the applied motion encoding gradients (MEGₓ, MEG_{y}, MEG_{z}).

5. Method according to claim 4, wherein a result from the reconstruction is used for evaluating stiffness and/or viscosity of an object being examined by the Magnetic Resonance Elastography.

6. Method according to one of the preceding claims, wherein the natural number of vibration periods within one sampling period (wp1, wp2, wp3, wp4) is equal to one, two or three.

7. Method according to one of the preceding claims, wherein the fixed delay (13) corresponds to one third, one quarter or one fifth of the vibration period,

8. Method according to one of the preceding claims, wherein the sampling period (wp1, wp2, wp3, wp4) comprises four time slots (12), one of the four time slots (12) for each of the three motion encoding gradients (MEGₓ, MEG_{y}, MEG_{z})and one time slot for a reference scan.

9. Method according to claim 8, wherein the four time slots (12) are the same size.

10. Method according to one of the preceding claims, wherein the three motion encoding gradients (MEGₓ, MEG_{y}, MEG_{z}) represent gradients in three orthogonal space directions.

11. Method according to one of the preceding claims, wherein no motion encoding gradient (MEGₓ, MEG_{y}, MEG_{z}) is applied during the reference scan.

12. Method according to one of the preceding claims, the method being repeated as k-space point acquisition step for each k-space point of a k-space.

13. Method according to claim 12, wherein the k-space point acquisition steps are synchronized with the vibration signal (11).

14. Magnetic Resonance Elastography device comprising
- signal generating means (6) for providing a periodical vibration signal (11) for exciting mechanical vibrations with a vibration period,
**characterized by**
- sampling means for sampling the vibration signal with a sampling period (wp1, wp2, wp3, wp4), the sampling period (wp1, wp2, wp3, wp4) corresponding to a natural number including zero of vibration periods plus a fixed time delay (13), wherein the fixed time delay (13) multiplied with a sampling number is equal to the vibration period, the sampling number being a natural number greater than two, and
- gradient application means for applying in each sampling period (wp1, wp2, wp3, wp4): one motion encoding gradient (MEGₓ, MEG_{y}, MEG_{z}) each in three motion encoding scans and no motion encoding gradient in one reference scan.

15. Computer program comprising instructions which, when the program is executed by a Magnetic Resonance Elastography device according to claim 14, cause the Magnetic Resonance Elastography device to carry out the method of one of the claims 1 to 13.
